# EUROPEAN PATENT SPECIFICATION

(11) **EP 1 882 700 B1**
(45) Date of publication and mention of the grant of the patent: **26.01.2011**
(21) Application number: 07101561.4
(22) Date of filing: 01.02.2007
(51) Int. Cl.: C07K 14/74, C07K 1/13, C07K 17/02, C07K 19/00, G01N 33/532, G01N 33/566

(54) **Biotinylated MHC complexes and their uses**
Biotinylierte MHC-Komplexe und ihre Verwendungen
Complexes majeurs d'histocompatibilite (MHC) biotinylés et leurs utilisations

(30) Priority: 25.07.2006 GB 0614735
(43) Date of publication of application: 30.01.2008
(73) Proprietor: Prolmmune Limited, Oxford OX4 4GA (GB)
(72) Inventor: Schwabe, Nikolai Franz Gregor, Oxford OX2 6BP (GB); Napper, Catherine Elizabeth, Witney OX28 3UD (GB)
(74) Representative: Cremer & Cremer

(56) References cited:
- WO-A-96/26962
- GB-A- 2 424 645
- US-A1- 2003 017 447
- YANG JUNBAO ET AL: "In vivo biotinylation of the major histocompatibility complex (MHC) class II/peptide complex by coexpression of B irA enzyme for the generation of MHC class II/tetramers" HUMAN IMMUNOLOGY, vol. 65, no. 7, July 2004 (2004-07), pages 692-699, XP002455388 ISSN: 0198-8859
- KAWANA-TACHIKAWA AI ET AL: "An efficient and versatile mammalian viral vector system for major histocompatibility complex class I/peptide complexes" JOURNAL OF VIROLOGY, THE AMERICAN SOCIETY FOR MICROBIOLOGY, US, vol. 76, no. 23, December 2002 (2002-12), pages 11982-11988, XP002355241 ISSN: 0022-538X
- BECKETT D ET AL: "A minimal peptide substrate in biotin holoenzyme synthetase-catalyzed biotinylation" PROTEIN SCIENCE, CAMBRIDGE UNIVERSITY PRESS, CAMBRIDGE, GB, vol. 8, no. 4, 1999, pages 921-929, XP002971557 ISSN: 0961-8368
- JANK M M ET AL: "Expression and Biotinylation of a Mutant of the Transcarboxylase Carrier Protein from Propioni shermanii" PROTEIN EXPRESSION AND PURIFICATION, ACADEMIC PRESS, SAN DIEGO, CA, US, vol. 17, no. 1, October 1999 (1999-10), pages 123-127, XP004441634 ISSN: 1046-5928

## Description

### Filed of the Invention

The invention relates to biotinylated MHC complexes and their uses.

### Background

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells. They consist of alpha and beta chains, and a peptide bound in a groove formed by these chains when properly folded.

It has been established that isolated or recombinant forms of MHC-peptide molecules are useful for detecting, separating and manipulating T cells according to the specific peptide antigens these T cells recognise.

European Patent Application EP 812 331 (WO 96/26962) discloses a multimeric binding complex for labeling, detecting and separating mammalian T cells according to their antigen receptor specificity, the complex having the formula (α-β-P)ₙ, wherein (α-β-P) is an MHC peptide molecule, n is ≥ 2, α comprises an α chain of a MHC I or MHC II class molecule, β comprises a β chain of an MHC protein and P is a substantially homogeneous peptide antigen. The MHC peptide molecule is multimerised by biotinylating the C terminus of one of the α or β chain of the MHC molecule and coupling of MHC monomers to tetravalent streptavidin/avidin or by providing a chimeric protein of an MHC molecule which is modified at the C terminus of one of the α or β chain to comprise an epitope which is recognised by a corresponding antibody that serves as a multimerising entity. The document further teaches use of the MHC oligomers for detecting, labeling and separating specific T cells according to their TCR specificity.

In one embodiment EP812331 describes a biotinylated version of an MHC monomer whereby a biotinylation peptide is fused to the α or β chain of the MHC monomer and which can be biotinylated with a biotinylating enzyme. The sequence for the biotinylation peptide used in an example is disclosed in EP711303.

EP711303 discloses biotinylation peptides of less than 50 amino acids in length and more specifically it discloses a group of mimetic peptides that can be as short as 14 amino acids in length, which mimic the function of naturally occurring biotinylation peptides that are usually significantly longer than 50 amino acids in length. While the short mimetic peptides of EP711303 approximate the conformation of the enzyme recognition site of a natural biotinylation peptide, they do not form the globular domain structure that is typical for naturally occurring biotinylation peptides.

US5,252,466 describes fusion proteins having a site for *in vivo* post translational modifications and methods for making and purifying them. More specifically US5,252,466 discloses fusion proteins, which can be biotinylated on a natural biotinylation domain with a biotinylating enzyme *in vivo.*

US2003/0017447A1 discloses the use of recombinant biotinylated MHC molecules in the detection of anti-HLA antibodies in serum samples from prospective transplant recipients, whereby anti-HLA antibody activity can be determined against a single MHC allele at a time. In an example US2003/0017447A1 uses monomeric recombinant MHC-peptide complexes for this purpose, which have been made similarly to those of EP812,331 using the short biotinylation peptides of EP711,303 fused to the MHC alpha chain of Class I MHC molecules.

In summary biotinylated MHC-peptide complexes have been used in the past for a variety of purposes. Two main applications include:
1) use of such complexes in forming multimeric and specifically tetrameric MHC-peptide complexes for detecting and separating antigen specific T cells, as seen in EP812331; and
2) use of such complexes in detecting anti-HLA serum antibodies in serum samples from prospective organ recipients, as seen in US2003/0017447.

In both cases in the past biotinylated MHC peptide complexes have been made by fusing a short mimetic biotinylation peptide as described in EP711303 to the C-terminus of the MHC alpha or chain.

The biotinylation peptide can then be biotinylated with a biotinylating enzyme, such as biotin-protein ligase BirA.

### The Invention

The invention demonstrates an improved choice of biotinylation peptide to be used in a combination or fusion with an MHC molecule for immobilizing or multimerising such MHC molecules for a variety of purposes, such as the applications described in EP812331 and US2003/0017447A1.

Although the length of the short mimetic biotinylation peptide was heralded as an advantage over longer naturally occurring sequences it can be advantageous to use a larger biotinylation domain in order to obtain better spacing of the biotinylated residue in the fusion protein from the MHC molecule while maintaining a rigid and defined structure of the folded protein complex.

We have shown for the first time that the fusion between an MHC complex and a naturally occurring biotinylation domain larger than 50 amino acids, such as the biotinyl carboxyl carrier protein (BCCP) subunit of acetyl CoA carboxylase in *E.coli.,* can be used to generate biotinylated MHC complexes. Such complexes and multimers thereof are useful for detecting antigen specific T cells in flow cytometry and also for detecting anti-HLA serum antibodies in the sera of transplant recipients. In this transplantation context it is believed that the extra spacing between the biotinylation site of the fused MHC complexes of the invention and the functional MHC portion of such complexes can provide an improvement of the recognition of certain epitopes located on the functional MHC portion by anti-MHC antibodies.

We have found that the biotinylated MHC complexes can be generated easily with known synthesis methods, be produced at good yields compared to the complexes of the prior art and be biotinylated with high efficiency.

In its first aspect thereof the present invention comprises a chimeric peptide comprising an MHC peptide and a biotinylation peptide which either is a natural biotinylation peptide or has a greater than 70% sequence homology to a natural biotinylation peptide, wherein the biotinylation peptide has a minimal sequence required for being biotinylated that is longer than 50 amino acids in length.

In one embodiment the MHC peptide is a Class I MHC peptide.

In an alternative embodiment the MHC peptides is a Class II MHC peptide.

In a further embodiment the biotinylation peptide is biotinylated.

Preferably the biotinylation peptide is located in the chimeric protein after the C-terminal end of the MHC peptide.

In one embodiment the MHC peptide and the biotinylation peptide are separated from one another by a linker sequence.

In one embodiment the biotinylation peptide is selected from the group consisting of the biotinylation domain of BCCP and *Propionibacterium shermanii* 1.3S subunit of transcarboxilase.

In a third aspect thereof the present invention comprises an MHC peptide complex with the formula (α-β-P), wherein α comprises an α chain of a MHC I or MHC II class molecule, β comprises an β chain of a MHC I or MHC II class molecule, and P is a peptide antigen bound in the binding groove of the MHC molecule, wherein said MHC peptide complex comprises a chimeric protein of the invention.

In one embodiment peptide antigen P bound in the binding groove is substantially homogeneous.

In a fourth aspect thereof the present invention comprises a multimeric binding complex having the formula (α-β-P)ₙ, wherein (α-β-P) is an MHC peptide complex of the present invention, and wherein n≥2.

In one embodiment n=4.

In one embodiment the MHC peptide complexes are biotinylated and the multimeric binding complex is formed by binding the biotinylated MHC peptide complexes to a multivalent entity that binds to biotin with high affinity.

In this context "high affinity" means that the binding interaction typically subsists for more than 30 minutes and preferably for several hours.

In a further embodiment the multivalent entity is an avidin family protein and more preferably streptavidin.

In yet a further embodiment the multimeric binding complex comprises a label.

In a fifth aspect thereof the present invention comprises a method of labelling and or detecting mammalian T cells according to the specificity of their antigen receptor, the method comprising combining a multimeric binding complex according to the invention and a suspension or biological sample comprising T cells, and detecting the presence of specific binding of said complex and at least one of the T cells.

In a sixth aspect thereof the present invention comprises a method of separating mammalian T cells according to the specificity of their antigen receptor, the method comprising combining a multimeric binding complex according to the invention and a suspension or biological sample comprising T cells, and separating one or more T cells bound to said complex from unbound cells.

In a seventh aspect thereof the present invention comprises a method of detecting the presence of one or more anti-MHC antibodies in a sample comprising contacting said sample with at least one MHC complex of the invention or at least one multimeric binding complex of the invention and detecting the binding or absence of binding of the one or more anti-MHC antibodies to either said MHC complex(es) or multimeric binding complex(es).

In one embodiment the antibodies which are detected are IgG, IgM or IgA.

In another embodiment the peptide antigen P is derived from an antigen that occurs in less than 5% of a population group.

"Population group" in this context shall mean a group of individuals from the general population, which may or may not be restricted to a specific region, which may be subjected to testing of fluid or tissue samples with one or more of the MHC complexes, multimeric binding complexes and/or methods of the invention.

In another embodiment the MHC complex(es) is(are) attached to a solid support.

In another embodiment the MHC complex(es) is(are) biotinylated and immobilized to the support through binding to an avidin family protein which is itself bound to the solid support.

In another embodiment the avidin family protein is streptavidin.

In another embodiment said solid support is a spherical bead.

In another embodiment the bead comprises a detectable label.

In an alternative embodiment the solid support is a nitrocellulose strip.

In another alternative embodiment the solid support is an ELISA plate.

In one embodiment the MHC complex(es) is(are) synthesized in a prokaryotic expression system.

In one embodiment the sample is a body fluid sample.

In one embodiment the bound antibody or absence thereof is detected via an immunosorbent assay using an antibody conjugated to a signalling means.

In one embodiment a single solid support is carrying two or more different ones of the MHC complexes or of the multimeric binding complexes at discrete locations on said solid support.

In an alternative embodiment two or more different ones of the MHC complexes or of the multimeric binding complexes are immobilized on different ones of said solid supports.

In an eighth aspect thereof the present invention comprises a method for determining the suitability of an organ to be transplanted for a transplant recipient, comprising the method of the seventh aspect of the invention, wherein the sample is a serum sample of the prospective transplant recipient and the presence of antibodies in the recipient that are reactive to at least one MHC molecule in the organ are detected and at least one MHC allele is determined against which such antibodies are reactive.

In a ninth aspect thereof the present invention comprises a method for determining a rejection reaction against a transplanted organ comprising the method the seventh aspect of the invention, wherein the sample is a serum sample of the transplant recipient by detecting the presence of antibodies in the recipient that are reactive to at least one MHC molecule in the organ are detected and at least one MHC allele is determined against which such antibodies are reactive

In a tenth aspect thereof the invention comprises a method of depleting a sample of anti-MHC molecule antibodies comprising at least the steps of contacting said sample with at least one MHC complex of the invention or at least one multimeric binding complex of the invention optionally attached to a solid support, and removing at least the MHC complex(es) or multimeric binding complexes from the sample to which at least one anti-MHC antibody contained within the sample has bound.

In a eleventh aspect thereof the invention comprises a kit comprising at least the following components: a) one or more recombinant MHC complexes of the invention or at least one multimeric binding complex of the invention; b) optionally a solid support, together with means for attachment of the MHC complex(es) or the multimeric binding complex(es); and c) a means for detecting anti-MHC-antibodies, preferably an antibody which binds to the complex formed between said MHC complex(es) or multimeric binding complex(es) and naturally occurring antibodies to said molecules.

In a twelfth embodiment thereof the invention comprises methods for biotinylating a chimeric peptide as follows:

One embodiment comprises a method for biotinylating a chimeric peptide, the chimeric peptide comprising an MHC peptide and a biotinylation peptide which either is a natural biotinylation peptide or has a greater than 70% sequence homology to a natural biotinylation peptide, wherein the biotinylation peptide has a minimal sequence required for being biotinylated that is longer than 50 amino acids in length, wherein the chimeric peptide is incubated in a reaction mixture comprising biotin or a biotin analogue and a biotinylating enzyme, resulting in the biotinylation of the chimeric peptide.

Another embodiment comprises a method for biotinylating a chimeric peptide, the chimeric peptide comprising an MHC peptide and a biotinylation peptide which is biotinylation peptide is either is a natural biotinylation peptide or has a greater than 70% sequence homology to a natural biotinylation peptide, wherein the biotinylation peptide has a minimal sequence required for being biotinylated that is longer than 50 amino acids in length, the method comprising (i) constructing a recombinant DNA expression vector that encodes the chimeric peptide, (ii) transforming a recombinant host cell with said vector, and (iii) culturing said host cell in the presence of biotin or a biotin analogue and under conditions such that said fusion protein and a biotinylating enzyme are expressed, resulting in the biotinylation of said chimeric peptide.

Another embodiment comprises a method for biotinylating a chimeric peptide, the chimeric peptide comprising an MHC peptide and a biotinylation peptide which biotinylation peptide has a minimal sequence required for being biotinylated that is longer than 50 amino acids in length wherein the chimeric peptide is incubated in a reaction mixture comprising biotin or a biotin analogue and a biotinylating enzyme, resulting in the biotinylation of the chimeric peptide.

Another embodiment comprises a method for biotinylating a chimeric peptide, the chimeric peptide comprising an MHC peptide and a biotinylation peptide which biotinylation peptide has a minimal sequence required for being biotinylated that is longer than 50 amino acids in length the method comprising (i) constructing a recombinant DNA expression vector that encodes the chimeric peptide, (ii) transforming a recombinant host cell with said vector, and (iii) culturing said host cell in the presence of biotin or a biotin analogue and under conditions such that said fusion protein and a biotinylating enzyme are expressed, resulting in the biotinylation of said chimeric peptide.

The functional monomeric MHC complexes of the invention will usually be soluble isolated or recombinant MHC complexes that may be derived from MHC class I or class II complexes, preferably the extra-cellular part of an MHC class I complex or the extra-cellular part of an MHC class II complex. Each of these complexes consists of an MHC α chain and an MHC β chain. The functional complex(es) may further comprise a peptide antigen P bound in the binding groove formed by the MHC α and β chains.

The MHC complexes may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2 -microglobulin. Included in the murine H-2 subunits are the class I H-2K, H-2D, H-2L, and the class II I-Aα, I-Aβ, I-Eα and I-Eβ, and β2-microglobulin. Amino acid sequences of some representative MHC proteins are referenced in EP 812 331. In one embodiment the MHC complexes will be from classical MHC molecules, including from classical Class I and Class II MHC molecules. Also included in the scope of this invention are non-classical examples such as HLA-E, HLA-F, HLA-G, Qa1, and CD1. The CD1 monomer may instead of the peptide have a lipid bound in its binding groove. The present invention is also applicable to the situation where a lipid instead of a peptide is bound and the skilled worker will be capable of translating the above oligomerisation protocols to this situation.

In a preferred embodiment, the MHC peptide chains correspond to the soluble form of the normally membrane-bound protein. For class I subunits, the soluble form is derived from the native form by deletion of the transmembrane and cytoplasmic domains. For class I proteins, the soluble form will include the α1, α2 and α3 domains of the α chain. For class II proteins the soluble form will include the α1 and α2 or β1 and β2 domains of the α chain or β chain, respectively.

Not more than about 10, usually not more than about 5, preferably none of the amino acids of the transmembrane domain will be included. The deletion may extend as much as about 10 amino acids into the α3 domain. Preferably none of the amino acids of the α3 domain will be deleted. The deletion will be such that it does not interfere with the ability of the α3 domain to fold into a functional disulfide bonded structure. The class I β chain, β2m, lacks a transmembrane domain in its native form, and does not have to be truncated. Generally, no class II subunits will be used in conjunction with class I subunits.

The above deletion is likewise applicable to class II subunits. It may extend as much as about 10 amino acids into the α2 or β2 domain, preferably none of the amino acids of the α2 or β2 domain will be deleted. The deletion will be such that it does not interfere with the ability of the α2 or β2 domain to fold into a functional disulfide bonded structure. In addition, one may wish to substitute one or more amino acids with a different amino acid for similar reasons, usually not substituting more than about five amino acids in any one domain.

A natural biotinylation peptide within the scope of the present invention means a peptide derived from a biotinylation domain, that is occurring naturally in an organism and that is capable of being biotinylated by a corresponding biotinylating enzyme. The biotinylation peptide will be derived in a manner such that it retains its property of being biotinylated by a corresponding biotinylating enzyme and have a greater than 70%, preferably greater than 80%, and more preferably greater than 90% sequence homology with a segment of the naturally occurring biotinylation domain that is also still capable of being biotinylated by a corresponding biotinylating enzyme. For example the biotinylation peptide may be derived by selecting the minimal sequence from the naturally occurring biotinylation domain that can be biotinylated by a corresponding enzyme.

Examples for the natural biotinylation peptide include the biotinyl carboxyl carrier protein (BCCP) subunit of acetyl CoA carboxylase. The corresponding biotinylation reaction is catalyzed by the biotin-protein ligase (BirA), the product of the birA gene (US5,252,466). Another example for a natural biotinylation peptide is the 1.3S subunit of transcarboxilase in *Propionibacterium shermanii.*

All natural biotinylation domains known to date have minimally required sequences for being biotinylated by a biotinylating enzyme that are longer than 50 amino acids in length (EP711303).

Other suitable natural biotinylation peptides can be derived from carboxylase proteins that are capable of being biotinylated from such species as, e.g., *Homo sapiens,* Chicken, *E. coli.,* Tomato, *S*. *cerevisiae.*

Typically the minimal or near minimal sequence required for successful enzymatic biotinylation of such proteins would be determined and used in constructing the chimeric peptides of the invention.

The biotinylation peptides of the invention can be fused to any of the termini of the MHC alpha or beta chains, but preferably such a fusion will be made on the C-terminus of one of the chains.

A flexible or rigid linker sequence may be interposed between the MHC peptide and the natural biotinylation peptide in the chimeric protein of the invention to allow for the formation of monomeric MHC complexes that have desired structural properties. Flexible linkers include glycine-serine linkers and rigid linkers include protein sequences that form soluble alpha helical conformations.

The chimeric protein of the invention may further comprise additional amino acid sequences that enable labelling or purification on either one of its polypeptide termini. Equally the MHC complexes of the invention may have such labelling or purification sequences on either one of the termini of the MHC peptide chain which is not a chimeric peptide of the invention.

In the past it has been mentioned that natural biotinylation domains are subject to enzymatic degradation, e.g. during soluble or periplasmic expression. The inventors have found that this issue can be resolved by expressing the fusion proteins in a prokaryotic expression system in inclusion bodies, which reduces protease contamination. The resulting protein denatured can then be purified in a number of repeated wash steps using a detergent wash buffer.

Functional MHC complexes can then be formed by refolding of the complexes using methods well known in the art. It was found that this process ensures that the fused domain stays in tact, especially after the complexes of the invention have undergone proper chromatographic purification.

Where the biotinylation of the chimeric protein is carried out during or following expression of the chimeric protein and during cell culture, expression and culture methods analogous to those described in EP711303 or US5,252,466 can be followed to achieve biotinylation.

### Example:

A chimeric protein (SEQ ID NO: 1) is generated by fusing (in N-terminal to C-terminal direction) (i) amino acids 25-300 of the unprocessed HLA-A*0201 alpha (heavy) chain precursor protein (SEQ ID NO: 2) followed by (ii) one glycine and one serine residue followed by (iii) amino acids 81-156 unprocessed precursor of the BCCP protein of acetyl-CoA carboxylase of *E.coli* strain K12 (SEQ ID NO: 3). The fusion protein is generated using molecular cloning techniques well known in the art. Purified HLA heavy chain can be obtained by expression in inclusion bodies in *E. coli.* using a suitable expression system, such as the pET system (Novagen, Milwaukee, WI, USA). Recombinant biotinylated MHC peptide complexes can be generated according to US2003/0017447A1. Specifically, native HLA-A2 monomeric MHC peptide complexes are refolded from denatured MHC alpha and human beta-2-microglobulin in the presence of the peptide GLCTLVAML (EBV BMLF-1 280-288; SEQ ID NO: 4). This peptide is known to bind strongly to HLA-A2 and is an immunodominant T cell epitope from Epstein Barr Virus (EBV). Refolded complexes are biotinylated overnight with the enzyme BirA as described in US2003/0017447A1. Biotinylated complexes are purified by size exclusion chromatography and the -50kD peak is recovered. The recovered material may be subjected to a second chromatography step, such as ion exchange chromatography, if desired, or may simply be concentrated to a suitable protein concentration appropriate for further use. The protein concentration is determined by the method of Bradford and the level of biotinylation can be determined, e.g. via the EZ^{™} Biotin Quantitation Kit (Pierce Biotechnology, Inc., Rockford, IL, USA).

For use in detecting antigen specific T cells, the biotinylated MHC complexes of the invention can be conjugated in a 4:1 molar ration to fluorescent labelled streptavidin, such as streptavidin:PE (Molecular Probes, Eugene, OR, USA). The complexes can then be used e.g. in flow cytometry to detect antigen-specific T cells as described in EP812331.

Similarly, for use in detecting anti-HLA antibodies the complexes can be plated into streptavidin-coated ELISA plates using a single-specificity HLA molecule per well (HLA complexes of a single allele in one well, HLA complexes of different alleles in different wells).

A typical ELISA set up would be as follows:

Coat wells with 100 µl Streptavidin (1ng/µl; 1:1000 dilution of 1mg/ml stock) in Coating buffer (0.1 M NaHCO₃, pH8.3) and leave at 4°C overnight. Wash plates 4 times with phosphate buffered saline (PBS)-Tween® (0.1%). Add 200 µl of Blocking buffer (5% bovine serum albumin (BSA)/PBS + 5% Glycine) and leave at room temperature for 1 hour. Wash plates 4 x PBS-Tween® (0.1%). Add 100 µl MHC monomers (0.5ng/µl) in coating buffer to each well (single MHC allele per well) and leave at room temperature for 1 hour. Wash plates 4 x PBS-Tween® (0.1%). Add 50 µl of serum (1:10 dilution) in PBS to each well and leave at room temperature for 1 hour. Wash plates 4 x PBS-Tween® (0.1%). Add 100 µl Rabbit anti Human IgA, G, M, kappa, lambda-horseradish peroxidase (HRP) (1:5000 dilution) in PBS and incubate at room temperature on the shaker at 250 rpm for 1 hour. Wash plates 4 x PBS-Tween® (0.1%). Add 50 µl tetramethylbenzidine (TMB) and leave it for 10 minutes. Stop reaction with 50 µl H₂SO₄ and measure OD₄₅₀.

### APPENDIX

### SEQUENCE LISTING

SEQ ID NO: 1
   A2BCCP Chimeric Protein
   Origin: Artificial
SEQ ID NO: 2
   HLA class histocompatibility antigen, A-2 alpha chain precursor Origin: Human
SEQ ID NO: 3
   Biotin carboxyl carrier protein of acetyl-CoA carboxylase
   Origin *E.coli,* strain K12
SEQ ID NO: 4
   Epstein Barr Virus BMLF-1 protein amino acids 280-288
   Origin: Epstein Barr Virus
   1 GLCTLVAML

## Claims

1. A chimeric peptide comprising an MHC peptide and a biotinylation peptide which either is a natural biotinylation peptide or has a greater than 70% sequence homology to a natural biotinylation peptide, wherein the biotinylation peptide has a minimal sequence required for being biotinylated that is longer than 50 amino acids in length.

2. A chimeric peptide of claim 1 wherein the MHC peptide is a Class I MHC peptide.

3. A chimeric peptide of claim 1 wherein the MHC peptide is a Class II MHC peptide.

4. A chimeric peptide of any preceding claim wherein the biotinylation peptide is biotinylated.

5. A chimeric peptide of any preceding claim wherein the biotinylation peptide is located in the chimeric protein after the C-terminal end of the MHC peptide.

6. A chimeric peptide of any preceding claim wherein the MHC peptide and the biotinylation peptide are separated by a linker sequence.

7. A chimeric peptide of any preceeding claim wherein the biotinylation peptide is selected from the group consisting of the biotinylation domain of BCCP and *Propionibacterium shermanii* 1.3S subunit of transcarboxilase.

8. An MHC peptide complex with the formula (α-β-P), wherein a comprises an α, chain of a MHC I or MHC II class molecule, β comprises an β chain of a MHC I or MHC II class molecule, and P is a peptide antigen bound in the binding groove of the MHC molecule, wherein said MHC peptide complex comprises a chimeric protein of any preceding claim.

9. An MHC peptide complex of claim 8, wherein the peptide antigen P bound in the groove is substantially homogeneous.

10. A multimeric binding complex having the formula (α-β-P)ₙ, wherein (α-β-P) is an MHC peptide complex of claim 8 or claim 9, and wherein n≥2.

11. A multimeric binding complex of claim 10 wherein the MHC peptide complexes are biotinylated and the multimeric binding complex is formed by binding the biotinylated MHC peptide complexes to a multivalent entity that binds to biotin with high affinity.

12. A multimeric binding complex of claim 11 wherein the multivalent entity is an avidin family protein and preferably streptavidin.

13. A multimeric binding complex of claims 10-12 comprising a label.

14. A method of labelling and or detecting mammalian T cells according to the specificity of their antigen receptor, the method comprising
(i) combining a multimeric binding complex according to any one of claim 10-13 and a suspension or biological sample comprising T cells, and
(ii) detecting the presence of specific binding of said complex and at least one of the T cells.

15. A method of separating mammalian T cells according to the specificity of their antigen receptor, the method comprising
(i) combining a multimeric binding complex according to any one of claims 10-13 and a suspension or biological sample comprising T cells, and
(ii) separating one or more T cells bound to said complex from unbound cells.

16. A method of detecting the presence of one or more anti-MHC antibodies in a sample comprising contacting said sample with at least one MHC complex according to any of claims 8-9 or at least one multimeric binding complex of any of claims 10-13 and detecting the binding or absence of binding of the one or more anti-MHC antibodies to either the MHC complex(es) or the multimeric binding complex(es).

17. A method according to claim 16 wherein the antibodies which are detected are IgG, IgM or IgA.

18. A method according to claims 16-17 wherein the peptide antigen P is derived from an antigen that occurs in less than 5% of a population group..

19. A method according to any of claims 16-18 wherein the MHC complex(es) is(are) attached to a solid support.

20. A method according to claim 19 wherein the MHC complex(es) is(are) biotinylated and immobilized to the solid support through binding to an avidin family protein which is itself bound to the solid support.

21. A method of claim 20 wherein the avidin family protein is streptavidin.

22. A method according to claims 19-21 wherein said solid support is a spherical bead.

23. A method according to claim 22 wherein the bead comprises a detectable label.

24. The method according to claims 19-21 wherein said solid support is a nitrocellulose strip.

25. The method according to claims 19-21 wherein said solid support is an ELISA plate.

26. The method according to claims 16-25 wherein the MHC complex(es) is(are) synthesized in a prokaryotic expression system.

27. The method according to claims 16-26 wherein the sample is a body fluid sample.

28. The method according to claims 16-27 wherein the bound antibody or absence thereof is detected via an immunosorbent assay using an antibody conjugated to a signalling means.

29. A method according to claims 16-28 wherein a single solid support is carrying two or more different ones of the MHC complexes or of the multimeric binding complexes at discrete locations on said solid support.

30. A method according to claims 16-28 wherein two or more different ones of the MHC complexes or of the multimeric binding complexes are immobilized on a different ones of said solid supports.

31. A method for determining the suitability of an organ to be transplanted for a transplant recipient, comprising the method of any of claims 16-30, wherein the sample is a serum sample of the prospective transplant recipient and the presence of antibodies in the recipient that are reactive to at least one MHC molecule in the organ are detected and at least one MHC allele is determined against which such antibodies are reactive.

32. A method for determining a rejection reaction against a transplanted organ comprising the method of any of claims 16-30, wherein the sample is a serum sample of the transplant recipient and the presence of antibodies in the recipient that are reactive to at least one MHC molecule in the organ are detected and at least one MHC allele is determined against which such antibodies are reactive.

33. A method of depleting a sample of anti-MHC molecule antibodies comprising at least the steps of contacting said sample with at least one MHC complex of any of claims 8-9 or at least one multimeric binding complex of any of claims 10-13, optionally attached to a solid support, and removing at least the MHC complex or multimeric binding complex from the sample to which at least one anti-MHC antibody contained within the sample has bound.

34. A kit comprising at least the following components: a) one or more recombinant MHC complexes according to any of claims 8-9 or at least one multimeric binding complex of any of claims 10-13; b) optionally a solid support, together with means for attachment of the MHC complex(es) or the multimeric binding complex(es); and c) a means for detecting anti-MHC-antibodies.

35. A method for biotinylating a chimeric peptide, the chimeric peptide comprising an MHC peptide and a biotinylation peptide which either is a natural biotinylation peptide or has a greater than 70% sequence homology to a natural biotinylation peptide, wherein the biotinylation peptide has a minimal sequence required for being biotinylated that is longer than 50 amino acids in length, wherein the chimeric peptide is incubated in a reaction mixture comprising biotin or a biotin analogue and a biotinylating enzyme, resulting in the biotinylation of the chimeric peptide.

36. A method for biotinylating a chimeric peptide, the chimeric peptide comprising an MHC peptide and a biotinylation peptide which biotinylation peptide is either a natural biotinylation peptide or has a greater than 70% sequence homology to a natural biotinylation peptide, wherein the biotinylation peptide has a minimal sequence required for being biotinylated that is longer than 50 amino acids in length, the method comprising (i) constructing a recombinant DNA expression vector that encodes the chimeric peptide, (ii) transforming a recombinant host cell with said vector, and (iii) culturing said host cell in the presence of biotin or a biotin analogue and under conditions such that said fusion protein and a biotinylating enzyme are expressed, resulting in the biotinylation of said chimeric peptide.

37. A method for biotinylating a chimeric peptide, the chimeric peptide comprising an MHC peptide and a biotinylation peptide which biotinylation peptide has a minimal sequence required for being biotinylated that is longer than 50 amino acids in length wherein the chimeric peptide is incubated in a reaction mixture comprising biotin or a biotin analogue and a biotinylating enzyme, resulting in the biotinylation of the chimeric peptide.

38. A method for biotinylating a chimeric peptide, the chimeric peptide comprising an MHC peptide and a biotinylation peptide which biotinylation peptide has a minimal sequence required for being biotinylated that is longer than 50 amino acids in length the method comprising (i) constructing a recombinant DNA expression vector that encodes the chimeric peptide, (ii) transforming a recombinant host cell with said vector, and (iii) culturing said host cell in the presence of biotin or a biotin analogue and under conditions such that said fusion protein and a biotinylating enzyme are expressed, resulting in the biotinylation of said chimeric peptide.

## Patentansprüche

1. Chimäres Peptid, umfassend ein MHC-Peptid und ein Biotinylierungspeptid, das entweder ein natürliches Biotinylierungspeptid ist oder mit einem natürlichen Biotinylierungspeptid eine Sequenzhomologie von mehr als 70 % hat, worin das Biotinylierungspeptid eine Minimalsequenz aufweist, die erforderlich ist, um biotinyliert zu werden, die länger als 50 Aminosäuren lang ist.

2. Chimäres Peptid nach Patentanspruch 1, worin das MHC-Peptid ein MHC-Klasse-I-Peptid ist.

3. Chimäres Peptid nach Patentanspruch 1, worin das MHC-Peptid ein MHC-Klasse-II-Peptid ist.

4. Chimäres Peptid nach irgendeinem der vorstehenden Patentansprüche, worin das Biotinylierungspeptid biotinyliert ist.

5. Chimäres Peptid nach irgendeinem der vorstehenden Patentansprüche, worin das Biotinylierungspeptid in dem chimären Protein nach dem C-terminalen Ende des MHC-Peptids angeordnet ist.

6. Chimäres Peptid nach irgendeinem der vorstehenden Patentansprüche, worin das MHC-Peptid und das Biotinylierungspeptid durch eine Linkersequenz getrennt sind.

7. Chimäres Peptid nach irgendeinem der vorstehenden Patentansprüche, worin das Biotinylierungspeptid ausgewählt ist aus der Gruppe, bestehend aus der Biotinylierungsdomäne von BCCP und der 1.3S-Untereinheit der Transcarboxylase von Propionibacterium shermanii.

8. MHC-Peptidkomplex mit der Formel (α-β-P), worin α eine α-Kette eines MHC-Klasse-I- oder MHC-Klasse-II-Moleküls umfasst, β eine β-Kette eines MHC-Klasse-I- oder MHC-Klasse-II-Moleküls umfasst und P ein in der Bindungsgrube des MHC-Moleküls gebundenes Peptidantigen ist, worin dieser MHC-Peptidkomplex ein chimäres Protein nach irgendeinem der vorstehenden Patentansprüche umfasst.

9. MHC-Peptidkomplex nach Patentanspruch 8, worin das in der Grube gebundene Peptidantigen P im Wesentlichen homogen ist.

10. Multimerischer Bindungskomplex mit der Formel (α-β-P)ₙ, worin (α-β-P) ein MHC-Peptidkomplex nach Patentanspruch 8 oder Patentanspruch 9 ist und worin n ≥ 2 ist.

11. Multimerischer Bindungskomplex nach Patentanspruch 10, worin die MHC-Peptidkomplexe biotinyliert sind und der multimerische Bindungskomplex durch Bindung der biotinylierten MHC-Peptidkomplexe an eine mehrwertige Einheit, die an Biotin mit hoher Affinität bindet, gebildet ist.

12. Multimerischer Bindungskomplex nach Patentanspruch 11, worin die mehrwertige Einheit ein Protein der Avidinfamilie ist und vorzugsweise Streptavidin ist.

13. Multimerischer Bindungskomplex nach den Patentansprüchen 10-12, umfassend eine Markierung.

14. Verfahren zum Markieren und/oder zum Detektieren von Säuger-T-Zellen nach der Spezifität ihres Antigenrezeptors, wobei das Verfahren umfasst
(i) Mischen eines multimerischen Bindungskomplexes nach irgendeinem der Patentansprüche 10-13 mit einer Suspension oder biologischen Probe, die T-Zellen umfasst, und
(ii) Detektieren der Anwesenheit spezifischer Bindung von diesem Komplex und wenigstens einer der T-Zellen.

15. Verfahren zum Separieren von Säuger-T-Zellen nach der Spezifität ihres Antigenrezeptors, wobei das Verfahren umfasst
(i) Mischen eines multimerischen Bindungskomplexes nach irgendeinem der Patentansprüche 10-13 mit einer Suspension oder biologischen Probe, die T-Zellen umfasst, und
(ii) Separieren einer oder mehrerer T-Zellen, die an diesen Komplex gebunden sind, von ungebundenen Zellen.

16. Verfahren zum Detektieren der Anwesenheit eines oder mehrerer Anti-MHC-Antikörper in einer Probe, umfassend das In-Kontakt-Bringen dieser Probe mit wenigstens einem MHC-Komplex nach irgendeinem der Patentansprüche 8-9 oder mit wenigstens einem multimerischen Bindungskomplex nach irgendeinem der Patentansprüche 10-13 und Detektieren der Bindung oder der Abwesenheit von Bindung des einen oder der mehreren Anti-MHC-Antikörper/s an entweder den/die MHC-Komplex/e oder den/die multimerischen Bindungskomplexle.

17. Verfahren nach Patentanspruch 16, worin die Antikörper, die detektiert werden, IgG, IgM oder IgA sind.

18. Verfahren nach den Patentansprüchen 16-17, worin das Peptidantigen P abgeleitet ist aus einem Antigen, das in weniger als 5 % einer Bevölkerungsgruppe vorkommt.

19. Verfahren nach irgendeinem der Patentansprüche 16-18, worin der/die MHC-Komplex/e an einen festen Träger angeheftet ist/sind.

20. Verfahren nach Patentanspruch 19, worin der/die MHC-Komplex/e biotinyliert ist/sind und auf dem festen Träger immobilisiert ist/sind durch Bindung an ein Protein der Avidinfamilie, das wiederum selbst an den festen Träger gebunden ist.

21. Verfahren nach Patentanspruch 20, worin das Protein der Avidinfamilie Streptavidin ist.

22. Verfahren nach den Patentansprüchen 19-21, worin dieser feste Träger eine kugelförmige Perle ist.

23. Verfahren nach Patentanspruch 22, worin die Perle eine detektierbare Markierung umfasst.

24. Verfahren nach den Patentansprüchen 19-21, worin dieser feste Träger ein Nitrocellulosestreifen ist.

25. Verfahren nach den Patentansprüchen 19-21, worin dieser feste Träger eine ELISA-Platte ist.

26. Verfahren nach den Patentansprüchen 16-25, worin der/die MHC-Komplex/e in einem prokaryotischen Expressionssystem synthetisiert ist/sind.

27. Verfahren nach den Patentansprüchen 16-26, worin die Probe eine Körperflüssigkeitsprobe ist.

28. Verfahren nach den Patentansprüchen 16-27, worin der gebundene Antikörper oder seine Abwesenheit über einen Immunadsorptionstest detektiert wird unter Verwendung eines Antikörpers, der mit Signal gebenden Mitteln konjugiert ist.

29. Verfahren nach den Patentansprüchen 16-28, worin ein einzelner fester Träger zwei oder mehrere unterschiedliche MHC-Komplexe oder multimerische Bindungskomplexe an unterschiedlichen Örtlichkeiten auf diesem festen Träger trägt.

30. Verfahren nach den Patentansprüchen 16-28, worin zwei oder mehr unterschiedliche MHC-Komplexe oder multimerische Bindungskomplexe immobilisiert sind auf unterschiedlichen Exemplaren dieser festen Träger.

31. Verfahren zum Bestimmen der Geeignetheit eines zu transplantierenden Organs für einen Transplantatempfänger, umfassend das Verfahren nach irgendeinem der Patentansprüche 16-30, worin die Probe eine Serumprobe des potentiellen Transplantatempfängers ist und die Anwesenheit von Antikörpern im Empfänger, die auf wenigstens ein MHC-Molekül in dem Organ reagieren, detektiert wird und wenigstens ein MHC-Allel bestimmt wird, gegen welches solche Antikörper reagieren.

32. Verfahren zum Bestimmen einer Abstoßungsreaktion gegen ein transplantiertes Organ, umfassend das Verfahren nach irgendeinem der Patentansprüche 16-30, worin die Probe eine Serumprobe des Transplantatempfängers ist und die Anwesenheit von Antikörpern im Empfänger, die auf wenigstens ein MHC-Molekül in dem Organ reagieren, detektiert wird und wenigstens ein MHC-Allel bestimmt wird, gegen welches solche Antikörper reagieren.

33. Verfahren zur Abreicherung einer Probe an Anti-MHC-Molekül-Antikörpem, umfassend wenigstens die Schritte des Kontaktierens dieser Probe mit wenigstens einem MHC-Komplex nach irgendeinem der Patentansprüche 8-9 oder wenigstens einem multimerischen Bindungskomplex nach irgendeinem der Patentansprüche 10-13, der/die optional an einen festen Träger angeheftet ist/sind, und Entfernens von wenigstens dem MHC-Komplex oder dem multimerischen Bindungskomplex aus der Probe, an welche wenigstens ein in der Probe enthaltener Anti-MHC-Antikörper gebunden hat.

34. Kit, umfassend wenigstens die folgenden Bestandteile:
a) einen oder mehrere rekombinante MHC-Komplexe nach irgendeinem der Patentansprüche 8-9 oder wenigstens einen multimerischen Bindungskomplex nach irgendeinem der Patentansprüche 10-13;
b) wahlweise einen festen Träger, zusammen mit Mitteln zum Anheften des/der MHC-Komplexe/s oder des/der multimerischen Bindungkomplexe/s; und
c) ein Mittel zum Detektieren von Anti-MHC-Antikörpern.

35. Verfahren zum Biotinylieren eines chimären Peptids, wobei das chimäre Peptid ein MHC-Peptid und ein Biotinylierungspeptid, das entweder ein natürliches Biotinylierungspeptid ist oder mit einem natürlichen Biotinylierungspeptid eine Sequenzhomologie von mehr als 70 % hat, umfasst, worin das Biotinylierungspeptid eine Minimalsequenz aufweist, die erforderlich ist, um biotinyliert zu werden, welche länger als 50 Aminosäuren lang ist, worin das chimäre Peptid in einer Reaktionsmischung, die Biotin oder ein Biotinanalogon und ein Biotinylierungsenzym umfasst, inkubiert wird, was in der Biotinylierung des chimären Peptids resultiert.

36. Verfahren zum Biotinylieren eines chimären Peptids, wobei das chimäre Peptid ein MHC-Peptid und ein Biotinylierungspeptid umfasst, wobei das Biotinylierungspeptid entweder ein natürliches Biotinylierungspeptid ist oder mit einem natürlichen Biotinylierungspeptid eine Sequenzhomologie von mehr als 70 % hat, worin das Biotinylierungspeptid eine Minimalsequenz aufweist, die erforderlich ist, um biotinyliert zu werden, die länger als 50 Aminosäuren lang ist, wobei das Verfahren umfasst
(i) Konstruieren eines rekombinanten DNS-Expressionsvektors, der das chimäre Peptid kodiert,
(ii) Transformieren einer rekombinanten Wirtszelle mit diesem Vektor, und
(iii) Kultivieren dieser Wirtszelle in der Anwesenheit von Biotin oder einem Biotinanalogon und unter Bedingungen, so dass dieses Fusionsprotein und ein Biotinylierungsenzym exprimiert werden, was in der Biotinylierung dieses chimären Peptids resultiert.

37. Verfahren zum Biotinylieren eines chimären Peptids, wobei das chimäre Peptid ein MHC-Peptid und ein Biotinylierungspeptid umfasst, wobei das Biotinylierungspeptid eine Minimalsequenz aufweist, die erforderlich ist, um biotinyliert zu werden, welche länger als 50 Aminosäuren lang ist, worin das chimäre Peptid in einer Reaktionsmischung inkubiert wird, die Biotin oder ein Biotinanalogon und ein Biotinylierungsenzym umfasst, was in der Biotinylierung des chimären Peptids resultiert.

38. Verfahren zum Biotinylieren eines chimären Peptids, wobei das chimäre Peptid ein MHC-Peptid und ein Biotinylierungspeptid umfasst, wobei das Biotinylierungspeptid eine Minimalsequenz aufweist, die erforderlich ist, um biotinyliert zu werden, welche länger als 50 Aminosäuren lang ist, wobei das Verfahren umfasst
(i) das Konstruieren eines rekombinanten DNS-Expressionsvektors, welcher das chimäre Peptid kodiert,
(ii) Transformieren einer rekombinanten Wirtszelle mit diesem Vektor, und
(iii) Kultivieren dieser Wirtszelle in der Anwesenheit von Biotin oder einem Biotinanalogon und unter Bedingungen, so dass dieses Fusionsprotein und ein Biotinylierungsenzym exprimiert werden, was in der Biotinylierung dieses chimären Peptids resultiert.

## Revendications

1. Peptide chimérique comprenant un peptide CMH et un peptide de biotinylation qui est soit un peptide de biotinylation naturelle, soit qui a une homologie de séquence de plus de 70% avec un peptide de biotinylation naturelle, dans lequel le peptide de biotinylation a une séquence minimale requise pour être biotinylé qui est plus longue que 50 acides aminés en longueur.

2. Peptide chimérique selon la revendication 1 dans lequel le peptide CMH est un peptide CMH de classe I.

3. Peptide chimérique selon la revendication 1 dans lequel le peptide CMH est un peptide CMH de classe II.

4. Peptide chimérique selon l'une quelconque des revendications précédentes dans lequel le peptide de biotinylation est biotinylé.

5. Peptide chimérique selon l'une quelconque des revendications précédentes dans lequel le peptide de biotinylation est situé dans la protéine chimérique après la fin de la chaîne C du peptide CMH.

6. Peptide chimérique selon l'une quelconque des revendications précédentes dans lequel le peptide CMH et le peptide de biotinylation sont séparés par une séquence de liaison.

7. Peptide chimérique selon l'une quelconque des revendications précédentes dans lequel le peptide de biotinylation est sélectionné dans le groupe comprenant le domaine de biotinylation de la BCCP et une sous-unité 1,3S de la transcarboxylase de *Propionibacterium shermanii.*

8. Complexe de peptide CMH de formule (α-β-P) dans lequel α comprend une chaîne α d'une molécule CMH de classe I ou II, β comprend une chaîne β d'une molécule CMH de classe I ou II et P est un peptide antigénique lié dans le sillon de liaison de la molécule CMH, dans lequel ledit complexe de peptide CMH comprend une protéine chimérique d'une quelconque des revendications précédentes.

9. Complexe de peptide CMH selon la revendication 8 dans lequel le peptide antigénique P lié dans le sillon est substantiellement homogène.

10. Complexe liant multimérique ayant la formule (α-β-P)ₙ dans lequel (α-β-P) est un complexe de peptide CMH selon la revendication 8 ou la revendication 9 et dans lequel n ≥ 2.

11. Complexe liant multimérique selon la revendication 10 dans lequel les complexes de peptide CMH sont biotinylés et le complexe liant multimérique est formé en liant les complexes de peptide CMH biotinylés à une entité multivalente qui se lie à de la biotine avec une affinité élevée.

12. Complexe liant multimérique selon la revendication 11 dans lequel l'entité multivalente est une protéine de la famille de l'avidine et est de préférence la streptavidine.

13. Complexe liant multimérique selon les revendications 10 à 12 comprenant une étiquette.

14. Procédé d'étiquettage et/ou de détection de cellules T de mammifères selon la spécificité de leur récepteur d'antigène, le procédé comprenant
(i) la combinaison d'un complexe liant multimérique selon l'une quelconque des revendications 10 à 13 et une suspension ou un échantillon biologique comprenant des cellules T et
(ii) la détection de la présence d'une liaison spécifique dudit complexe et d'au moins l'une des cellules T.

15. Procédé de séparation des cellules T de mammifères selon la spécificité de leur récepteur d'antigène, le procédé comprenant
(i) la combinaison d'un complexe liant multimérique selon l'une quelconque des revendications 10 à 13 et une suspension ou un échantillon biologique comprenant des cellules T et
(ii) la séparation d'une ou de plusieurs cellules T liées audit complexe des cellules non liées.

16. Procédé de détection de la présence d'un ou de plusieurs anticorps anti-CMH dans un échantillon comprenant la mise en contact dudit échantillon avec au moins un complexe CMH selon l'une des revendications 8 et 9 ou au moins un complexe liant multimérique selon l'une quelconque des revendications 10 à 13 et de détection de la liaison ou de l'absence de liaison de l'un ou plusieurs anticorps anti-CMH à soit le(s) complexe(s) CMH, soit le(s) complexe(s) multimérique(s).

17. Procédé selon la revendication 16 dans lequel les anticorps qui sont détectés sont IgG, IgM ou IgA.

18. Procédé selon les revendications 16-17 dans lequel le peptide antigénique P est dérivé d'un antigène qui se trouve dans moins de 5 % d'un groupe de population.

19. Procédé selon l'une quelconque des revendications 16 à 18 dans lequel le(s) complexe(s) CMH est(sont) attaché(s) à un support solide.

20. Procédé selon la revendication 19 dans lequel le(s) complexe(s) CMH est(sont) biotinylé(s) et immobilisé(s) au support solide par liaison à une protéine de la famille de l'avidine qui est elle-même liée au support solide.

21. Procédé selon la revendication 20 dans lequel la protéine de la famille de l'avidine est la streptavidine.

22. Procédé selon les revendications 19 à 21 dans lequel ledit support solide est une bille sphérique.

23. Procédé selon la revendication 22 dans lequel la bille comprend une étiquette qui peut être détectée.

24. Procédé selon les revendications 19 à 21 dans lequel ledit support solide est une bande de nitrocellulose.

25. Procédé selon les revendications 19 à 21 dans lequel ledit support solide est une plaque ELISA.

26. Procédé selon les revendications 16 à 25 dans lequel le(s) complexe(s) CMH est(sont) synthétisé(s) dans un système d'expression procaryotique.

27. Procédé selon les revendications 16 à 26 dans lequel l'échantillon est un échantillon de fluide corporel.

28. Procédé selon les revendications 16 à 27 dans lequel l'anticorps lié ou l'absence de celui-ci est détectée par un test d'immuno-absorption qui utilise un anticorps conjugué à un moyen de signalisation.

29. Procédé selon les revendications 16 à 28 dans lequel un seul support solide porte deux ou plus des complexes CMH différents différents ou des complexes liants multimériques différents à des endroits distincts sur ledit support solide.

30. Procédé selon les revendications 16 à 28 dans lequel deux ou plus des complexes CMH différents ou des complexes liants multimériques différents sont immobilisés sur des supports desdits supports solides.

31. Procédé pour déterminer l'aptitude d'un organe à être transplanté pour un receveur de transplantation comprenant le procédé selon l'une quelconque des revendications 16 à 30 dans lequel l'échantillon est un échantillon de sérum du receveur prospectif de la transplantation et la présence d'anticorps dans le receveur qui sont réactifs à au moins une molécule de CMH dans l'organe sont détectés et au moins un allèle du CMH est déterminé contre lequel de tels anticorps sont réactifs.

32. Procédé pour déterminer une réaction de rejet à l'encontre d'un organe transplanté comprenant le procédé selon l'une quelconque des revendications 16 à 30 dans lequel l'échantillon est un échantillon de sérum du receveur de la transplantation et la présence d'anticorps dans le receveur qui sont réactifs à au moins une molécule de CMH dans l'organe sont détectés et au moins un allèle du CMH est déterminé contre lequel de tels anticorps sont réactifs.

33. Procédé pour appauvrir un échantillon d'anticorps anti-molécule de CMH comprenant au moins les étapes de mise en contact dudit échantillon avec au moins un complexe CMH selon l'une des revendications 8-9 ou au moins un complexe liant multimérique selon l'une quelconque des revendications 10 à 13, attachés en option à un support solide, et d'enlever au moins le complexe CMH ou le complexe liant multimérique de l'échantillon auquel au moins un anticorps anti-CMH contenu à l'intérieur de l'échantillon est lié.

34. Kit comprenant au moins les composants suivants : a) un ou plusieurs complexes CMH recombinants selon l'une quelconque des revendications 8-9 ou au moins un complexe liant multimérique de l'une quelconque des revendications 10 à 13; b) facultativement un support solide, ensemble avec des moyens pour attacher le(s) complexe(s) CMH ou le(s) complexe(s) liant(s) multimérique(s) et c) un moyen pour détecter des anticorps anti-CMH.

35. Procédé pour biotinyler un peptide chimérique, le peptide chimérique comprenant un peptide CMH et un peptide de biotinylation qui est soit un peptide de biotinylation naturelle, soit qui a une homologie de séquence de plus de 70% avec un peptide de biotinylation naturelle, dans lequel le peptide de biotinylation a une séquence minimale requise pour être biotinylé qui est plus longue que 50 acides aminés en longueur, dans lequel le peptide chimérique est incubé dans un mélange de réaction comprenant de la biotine ou un équivalent de la biotine et une enzyme de biotinylation, ce qui résulte en la biotinylation du peptide chimérique.

36. Procédé pour biotinyler un peptide chimérique, le peptide chimérique comprenant un peptide CMH et un peptide de biotinylation, lequel peptide de biotinylation est soit un peptide de biotinylation naturelle, soit qui a une homologie de séquence de plus de 70% avec un peptide de biotinylation naturelle, dans lequel le peptide de biotinylation a une séquence minimale requise pour être biotinylé qui est plus longue que 50 acides aminés en longueur, le procédé comprenant (i) la construction d'un vecteur d'expression ADN recombinant qui code le peptide chimérique, (ii) la transformation d'une cellule hôte recombinante avec ledit vecteur et (iii) la culture de ladite cellule hôte en présence de la biotine ou d'un équivalent de la biotine et dans des conditions telles que ladite protéine de fusion et une enzyme de biotinylation sont exprimées, ce qui résulte en la biotinylation du peptide chimérique.

37. Procédé pour biotinyler un peptide chimérique, le peptide chimérique comprenant un peptide CMH et un peptide de biotinylation, lequel peptide de biotinylation a une séquence minimale requise pour être biotinylé qui est plus longue que 50 acides aminés en longueur, dans lequel le peptide chimérique est incubé dans un mélange de réaction comprenant de la biotine ou un équivalent de la biotine et une enzyme de biotinylation, ce qui résulte en la biotinylation du peptide chimérique.

38. Procédé pour biotinyler un peptide chimérique, le peptide chimérique comprenant un peptide CMH et un peptide de biotinylation, lequel peptide de biotinylation a une séquence minimale requise pour être biotinylé qui est plus longue que 50 acides aminés en longueur, le procédé comprenant (i) la construction d'un vecteur d'expression ADN recombinant qui code le peptide chimérique, (ii) la transformation d'une cellule hôte recombinante avec ledit vecteur et (iii) la culture de ladite cellule hôte en présence de la biotine ou d'un équivalent de la biotine et dans des conditions telles que ladite protéine de fusion et une enzyme de biotinylation sont exprimées, ce qui résulte en la biotinylation du peptide chimérique.


### SEQUENCE LISTING

<110> ProImmune Limited
<120> Biotinylated MHC Complexes and their Uses
<130> PRO 07009 EP
<150> GB 0614735.9
   <151> 2006-07-25
<160> 4
<170> PatentIn version 3.3
<210> 1
   <211> 354
   <212> PRT
   <213> Artificial Sequence
<220>
   <223> A2BCCP chimeric protein
<400> 1
<210> 2
   <211> 365
   <212> PRT
   <213> Human
<400> 2
<210> 3
   <211> 156
   <212> PRT
   <213> E. coli, strain K12
<400> 3
   Met Asp Ile Arg Lys Ile Lys Lys Leu Ile Glu Leu Val Glu Glu Ser
   1               5                   10                  15
   Gly Ile Ser Glu Leu Glu Ile Ser Glu Gly Glu Glu Ser Val Arg Ile
               20                  25                  30
   Ser Arg Ala Ala Pro Ala Ala Ser Phe Pro Val Met Gln Gln Ala Tyr
           35                  40                  45
   Ala Ala Pro Met Met Gln Gln Pro Ala Gln Ser Asn Ala Ala Ala Pro
       50                  55                  60
   Ala Thr Val Pro Ser Met Glu Ala Pro Ala Ala Ala Glu Ile Ser Gly
   65                  70                  75                  80
   His Ile Val Arg Ser Pro Met Val Gly Thr Phe Tyr Arg Thr Pro Ser
                   85                  90                  95
   Pro Asp Ala Lys Ala Phe Ile Glu Val Gly Gln Lys Val Asn Val Gly
               100                 105                 110
   Asp Thr Leu Cys Ile Val Glu Ala Met Lys Met Met Asn Gln Ile Glu
           115                 120                 125
   Ala Asp Lys Ser Gly Thr Val Lys Ala Ile Leu Val Glu Ser Gly Gln
       130                 135                 140
   Pro Val Glu Phe Asp Glu Pro Leu Val Val Ile Glu
   145                 150                 155
<210> 4
   <211> 9
   <212> PRT
   <213> Epstein Barr Virus
<400> 4
   Gly Leu Cys Thr Leu Val Ala Met Leu
   1               5